(19) Europäisches Patentamt
European Patent Office
Office européen des brevets

(11) **EP 2 504 308 B1**

(12) **EUROPÄISCHE PATENTSCHRIFT**

(45) Veröffentlichungstag und Bekanntmachung des Hinweises auf die Patenterteilung:
**19.05.2021 Patentblatt 2021/20**

(21) Anmeldenummer: **10779022.2**

(22) Anmeldetag: **23.11.2010**

(51) Int Cl.:
*C07C 67/08* (2006.01)      *C07C 69/54* (2006.01)
*C08F 220/12* (2006.01)

(86) Internationale Anmeldenummer:
**PCT/EP2010/067986**

(87) Internationale Veröffentlichungsnummer:
**WO 2011/064190 (03.06.2011 Gazette 2011/22)**

(54) **VERFAHREN ZUR HERSTELLUNG VON (METH)ACRYLATEN VON C17-ALKOHOLGEMISCHEN**

PROCESS FOR THE PRODUCTION OF (METH)ACRYLATES OF C17-ALCOHOL MIXTURES

PROCEDE DE FABRICATION DE (METH)ACRYLATES AVEC D'UN MÉLANGE D'ALCOOLS C17

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.11.2009 DE 102009047228**
**27.11.2009 US 264704 P**

(43) Veröffentlichungstag der Anmeldung:
**03.10.2012 Patentblatt 2012/40**

(73) Patentinhaber: **BASF SE**
**67056 Ludwigshafen am Rhein (DE)**

(72) Erfinder:
• **BETTE, Virginie**
**68165 Mannheim (DE)**
• **PETZOLDT, Jochen**
**67273 Weisenheim am Berg (DE)**
• **BREITSCHEIDEL, Boris**
**67117 Limburgerhof (DE)**

(74) Vertreter: **BASF IP Association**
**BASF SE**
**G-FLP-C006**
**67056 Ludwigshafen (DE)**

(56) Entgegenhaltungen:
**EP-A2- 1 342 762      WO-A1-02/50014**
**WO-A1-02/055472      WO-A1-2009/106550**
**WO-A1-2009/124979**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft ein Verfahren zur diskontinuierlichen Herstellung von (Meth)acrylaten von $C_{17}$-Alkoholgemischen durch Veresterung von (Meth)acrylsäure mit $C_{17}$-Alkoholgemischen mit einem mittleren Verzweigungsgrad (Iso-Index) von 3,01 bis 3,5 und ihre Verwendung.

Der Begriff (Meth)acrylsäure steht in dieser Schrift verkürzend für Methacrylsäure und/oder Acrylsäure, (Meth)acrylsäureester für Methacrylsäureester und/oder Acrylsäureester bzw. (Meth)acrylat für Methacrylat und/oder Acrylat.

Die auf Basis von (Meth)acrylaten von $C_{17}$-Alkoholgemischen hergestellten Polymere beziehungsweise Copolymere sind in Form von Polymerdispersionen von großer wirtschaftlicher Bedeutung. Sie finden beispielsweise Anwendung als Klebstoffe, Anstrichmittel oder Textil-, Leder- und Papierhilfsmittel.

Die Herstellung von höher alkylierten (Meth)acrylaten durch säurekatalysierte Veresterung von (Meth)acrylsäure mit $C_8$-$C_{20}$-Monoalkoholen ist bereits bekannt und beispielsweise in WO 2002/050014 A1 und WO 2002/050015 A1 beschrieben.

Aus der deutschen Offenlegungsschrift DE 2 317 226 A1 ist ein Verfahren zur Herstellung von (Meth)acrylsäureestern aus einem Gemisch von $C_{10}$-$C_{18}$-Alkoholen durch Tikatalysierte Umesterung von (Meth)acrylsäure bekannt. Die Veresterung erfolgt demnach in Gegenwart einer Kombination aus 2,6-Ditert.-butylparakresol (TBK) und adsorptionsfähiger Kohle als Polymerisationsinhibitoren. Das als Katalysator eingesetzte Titanalkoholat wird im Zuge der erhöhten Temperatur hydrolysiert und zusammen mit der Kohle abfiltriert.

Die japanische Offenlegungsschrift JP 11-80082 A offenbart die Veresterung von (Meth)acrylsäure mit $C_8$-$C_{22}$-Alkoholen in Gegenwart von sauren Katalysatoren wie beispielsweise p-Toluolsulfonsäure und Methansulfonsäure.

Die Umesterung von Methylmethacrylat mit langkettigen $C_3$-$C_{20}$-Alkoholen wird in der japanischen Offenlegungsschrift JP 05 070404 beschrieben. Diese Umesterung erfolgt demnach in Gegenwart von Kalium-, Magnesium- oder Natriumoxid- oder -hydroxid als Katalysator.

Darüber hinaus ist die Veresterung von (Meth)acrylsäure mit geradkettigen, gesättigten oder ungesättigten $C_8$-$C_{22}$-Fettalkoholen beschrieben, wobei die langkettigen Fettalkohole vor der Veresterung durch Destillation über Natriumboranat gereinigt werden.

In keiner der genannten Schriften wir die Herstellung von (Meth)acrylaten von $C_{17}$-Alkoholen explizit offenbart. Insbesondere wird nicht die Verwendung eines $C_{17}$-Alkoholgemisches, das einen mittleren Verzweigungsgrad von 2,8 bis 3,7 aufweist, nicht offenbart. Der Verzweigungsgrad des $C_{17}$-Alkoholgemischs und infolgedessen auch der daraus resultierenden (Meth)acrylsäureester ist besonders für den Einsatz der (Meth)acrylsäureester als Monomere und Comonomere zur Herstellung von (Co)Polymerisaten für verschiedene technische Anwendungen von Bedeutung, da bevorzugt Monomere und Comonomer mit einem nicht zu hohen Verzweigungsgrad eingesetzt werden sollen. Stattdessen beziehen sich die vorgenannten Dokumente im Wesentlichen auf die Synthese von $C_{16}$-, $C_{18}$- und $C_{20}$-Alkyl(meth)acrylate.

Aufgabe der vorliegenden Erfindung was es daher, ein Verfahren zur Herstellung von (Meth)acrylaten von $C_{17}$-Alkoholgemischen, die einen nicht zu hohen mittleren Verzweigungsgrad aufweisen, zur Verfügung zu stellen, mit dem die (Meth)acrylate von $C_{17}$-Alkoholgemischen in hohen Ausbeuten und in hohen Reinheiten erhalten werden. Weiterhin sollen Produkte mit niedrigen Farbzahlen resultieren.

Die Aufgabe wurde gelöst durch ein Verfahren zur Herstellung von (Meth)acrylaten von $C_{17}$-Alkoholgemischen, wobei der Erstarrungspunkt der (Meth)acrylate bei Atmospärendruck unter 0 °C liegt, durch Umsetzung von (Meth)acrylsäure mit einem $C_{17}$-Alkoholgemisch in Gegenwart mindestens eines sauren Katalysators und mindestens eines Polymerisationsinhibitors und in Gegenwart eines Lösungsmittels, das mit Wasser ein Azeotrop bildet, wobei die Veresterung in einem Reaktor mit Umlaufverdampfer durchführt wird, das Azeotrop abdestilliert und kondensiert wird, das Kondensat in eine wässrige und eine organische Phase zerfällt, und wobei das $C_{17}$-Alkoholgemisch einen mittleren Verzweigungsgrad (Iso-Index) von 3,01 bis 3,5 und einen Gehalt an Alkoholen mit 17 Kohlenstoffatomen von mindestens 95 Gew.-% aufweist.

In dem erfindungsgemäßen Verfahren werden $C_{17}$-Alkoholgemische eingesetzt, die einen mittleren Verzweigungsgrad (Iso-Index) von 3,01 bis 3,5 aufweisen. Bevorzugt weisen die $C_{17}$-Alkoholgemische einen mittleren Verzweigungsgrad von 3,05 bis 3,4 auf. Ganz besonderes bevorzugt weisen die $C_{17}$-Alkoholgemische einen mittleren Verzweigungsgrad im Bereich von etwa 3,1 auf.

Der mittlere Verzweigungsgrad des $C_{17}$-Alkoholgemischs und infolgedessen auch der daraus resultierenden (Meth)acrylsäureester ist erfindungswesentlich, da für die Verwendung dieser (Meth)acrylsäureester als Monomere und Comonomere zur Herstellung von (Co)Polymerisaten für verschiedene technische Anwendungen ein nicht zu hoher mittlerer Verzweigungsgrad von Bedeutung ist.

Als Verzweigungsgrad im Sinne der Erfindung ist die Zahl der Methylgruppen in einem Molekül des Alkohols abzüglich 1 definiert. Der mittlere Verzweigungsgrad ist der statistische Mittelwert der Verzweigungsgrade der Moleküle einer Probe. Der mittlere Verzweigungsgrad kann wie folgt $^1$H-NMR-spektroskopisch ermittelt werden: Dazu wird eine Probe des Alkohols bzw. Alkoholgemisches zunächst einer Derivatisierung mit Trichloracetylisocyanat (TAI) unterzogen. Dabei werden die Alkohole in die Carbaminsäureester überführt. Die Signale der auf diese Weise veresterten primären Alkohole liegen bei $\delta$ = 4,7 bis 4,0 ppm, die der (soweit vorhanden) veresterten sekundären Alkohole bei etwa 5 ppm und in der Probe vorhandenes Wasser reagiert mit TAI zur Carbaminsäure ab. Alle Methyl-, Methylen- und Methinprotonen liegen im Bereich von 2,4 bis 0,4 ppm. Die Signale < 1 ppm sind dabei den Methylgruppen zugeordnet. Aus dem so erhalten Spektrum lässt sich der mittlere Verzweigungsgrad (Iso-Index) wie folgt berechnen:

$$\text{Iso-Index} = ((F(CH_3)/3) / (F(CH_2\text{-}OH)/2)) - 1$$

wobei $F(CH_3)$ für die den Methylprotonen entsprechende Signalfläche und $F(CH_2\text{-}OH)$ für die Signalfläche der Methylenprotonen in der $CH_2$-OH-Gruppe steht.

Das $C_{17}$-Alkoholgemisch weist dabei einen Gehalt an Alkoholen mit 17 Kohlenstoffatomen von mindestens 95 Gew.-%, bevorzugt mindestens 98 Gew.-%, insbesondere wenigstens 99 Gew.-%, bezogen auf das Gesamtgewicht des $C_{17}$-Alkoholgemischs, auf. Speziell handelt es sich um ein $C_{17}$-Alkoholgemisch, das im Wesentlichen (d.h. zu mehr als 99,5 Gew.-%, speziell zu mehr als 99,9 Gew.-%) aus Alkoholen mit 17 Kohlenstoffatomen besteht.

Zur Herstellung von derartigen $C_{17}$-Alkoholgemischen wird an dieser Stelle auf die Offenlegungsschrift WO 2009/124979 A1 und die darin zitierte Literatur verwiesen.

Vorteilhaft an den oben beschriebenen $C_{17}$-Alkoholgemischen ist, dass sie eine hohe Reinheit von mindestens 95 Gew.-% und einen mittleren Verzweigungsgrad von 3,01 bis 3,5 aufweisen. Durch das erfindungsgemäße Verfahren zur Herstellung von (Meth)acrylsäureestern werden daher ebenfalls $C_{17}$-Alkyl(meth)acrylate mit einer hohen Reinheit erhalten. Bisher kommerziell erhältliche $C_{17}$-Alkyl(meth)acrylate sind üblicherweise Mischungen von $C_{16}$- und $C_{18}$-Alkyl(meth)acrylaten. Infolgedessen können die Mischungs- und Isomerenverhältnisse in verschiedenen Chargen unterschiedlich stark ausfallen. Dies übte sich bisher negativ auf die Eigenschaften der daraus resultierenden (Co)Polymerisate aus.

Dabei ist besonders vorteilhaft der tiefe Erstarrungspunkt der nach dem erfindungsgemäßen Verfahren hergestellten (Meth)acrylsäureester von $C_{17}$-Alkoholgemischen. Aufgrund der hohen Reinheit und des konstanten Verzweigungsgrades liegt der Erstarrungspunkt (bei Atmosphärendruck) unter 0 °C, bevorzugt unter -20 °C und besonders bevorzugt unter -40 °C. Hingegen weisen die zuvor beschriebenen bisher kommerziell erhältlichen $C_{17}$-Alkyl(meth)acrylate einen Erstarrungspunkt (bei Atmosphärendruck) von über 0 °C auf.

Das erfindungsgemäße Verfahren ist vorteilhaft, da ein hoher Veresterungsgrad erreicht wird und hohe Ausbeuten erzielt werden. Weiterhin tritt keine wesentliche Polymerisatbildung bei der Veresterung oder Aufarbeitung auf, und das Endprodukt ist weitgehend farblos.

Das bei der Veresterung entstehende Wasser, das mit dem Lösungsmittel ein Azeotrop bildet, wird über eine dem Reaktor aufgesetzte Kolonne ausgeschleust und kondensiert.

Das anfallende Kondensat (Azeotrop) zerfällt in eine wässrige Phase, die ausgeschleust und vorteilhaft aufgearbeitet (Rückextraktion der enthaltenen Säure) wird und eine Lösungsmittelphase, die als Rücklauf in die Kolonne und gegebenenfalls teilweise in den Reaktor und/oder Verdampfer zurückgeführt wird, wie in der DE 199 41 136 A1 und DE 100 63 175 A1 beschrieben ist.

Eine Rückextraktion der enthaltenen (Meth)acrylsäure erfolgt bevorzugt mit dem verwendeten Lösungsmittel als Extraktionsmittel, beispielsweise mit Cyclohexan bei einer Temperatur zwischen 10 und 40 °C und einem Verhältnis von wässriger Phase zu Extraktionsmittel von 1 : 5 - 30, bevorzugt 1 : 10 - 20. Die im Extraktionsmittel enthaltene Säure kann bevorzugt direkt in die Veresterung geführt werden.

Nach Beendigung der Veresterung wird das heiße Reaktionsgemisch rasch abgekühlt und gegebenenfalls mit Lösungsmittel verdünnt.

Anschließend wird das Lösungsmittel vom Zielester destillativ abgetrennt.

Das erfindungsgemäße Verfahren besteht im Wesentlichen aus folgenden Stufen:

1) Veresterung

Die Veresterungsapparatur besteht aus einem Reaktor mit Umlaufverdampfer und einer aufgesetzten Destillationskolonne mit Kondensator und Phasentrenngefäß.

Beim Reaktor kann es sich beispielsweise um einen Reaktor mit Doppelwandheizung oder/und innenliegenden Heizschlangen handeln. Vorzugsweise wird ein Reaktor mit außenliegendem Wärmetauscher und Natur- oder Zwangsumlauf (unter Verwendung einer Pumpe) eingesetzt. Im Falle von Naturumlauf wird der Kreisstrom ohne mechanische Hilfsmittel bewerkstelligt.

Geeignete Umlaufverdampfer sind dem Fachmann bekannt und beispielsweise beschrieben in R. Billet, Verdampfertechnik, HTB-Verlag, Bibliographisches Institut Mannheim, 1965, 53. Beispiele für Umlaufverdampfer sind Rohrbündelwärmetauscher, Plattenwärmetauscher etc.

Selbstverständlich können im Umlauf auch mehrere Wärmetauscher vorhanden sein.

Die Destillationskolonne ist von an sich bekannter Bauart und weist die üblichen Einbauten auf. Als Kolonneneinbauten kommen prinzipiell alle gängigen Einbauten in Betracht, beispielsweise Böden, Packungen und/oder Schüttungen. Von den Böden sind Glockenböden, Siebböden, Ventilböden, Thormannböden und/oder Dual-Flow-Böden bevorzugt, von den Schüttungen sind solche mit Ringen, Wendeln, Sattelkörpern oder Geflechten bevorzugt.

In der Regel sind 5 bis 20 theoretische Böden ausreichend.

Der Kondensator und das Trenngefäß sind von herkömmlicher Bauart.

(Meth)acrylsäure und das $C_{17}$-Alkoholgemisch werden in der Regel in äquivalenten Mengen eingesetzt, es kann aber auch ein Unterschuss oder Überschuss an (Meth)acrylsäure verwendet werden.

Sowohl (Meth)acrylsäure als auch (Meth)acrylsäureester sind polymerisationsfähige Verbindungen. Daher ist bereits im Verfahrensschritt der Veresterung auf eine ausreichende Polymerisationsinhibierung zu achten. Geeignete Polymerisationsinhibitoren werden weiter unten offenbart. Von den dort genannten Stabilisatoren ist für die Veresterung insbesondere Kupfer(II)chlorid geeignet.

Vorzugsweise wird ein Überschuss an (Meth)acrylsäure pro zu veresternde Hydroxygruppe (Äquivalente) von 5 - 100 mol% eingestellt, bevorzugt 5 - 50 mol% und besonders bevorzugt 5 - 10 mol%.

Als Veresterungskatalysatoren kommen die üblichen Mineralsäuren und Sulfonsäuren in Frage, vor-zugsweise Schwefelsäure, Phosphorsäure, Alkylsulfon-säuren (z.B. Methansulfonsäure, Trifluormethansulfon-säure) und Arylsulfonsäuren (z.B. Benzol-, p-Toluol- oder Dodecylbenzolsulfonsäure) oder Gemische davon, aber auch saure Ionenaustauscher oder Zeolithe sind denkbar.

Besonders bevorzugt sind Schwefelsäure, Methansulfonsäure und p-Toluolsulfonsäure oder Gemische davon.

Sie werden in der Regel in einer Menge von 0,1 - 5 Gew.-%, bezogen auf das Veresterungsgemisch, eingesetzt, bevorzugt 0,5 - 5 und besonders bevorzugt 1 - 4 Gew.-%.

Falls erforderlich kann der Veresterungskatalysator aus dem Reaktionsgemisch mit Hilfe eines Ionenaustauschers entfernt werden. der Ionenaustauscher kann dabei direkt in das Reaktionsgemisch gegeben und anschließend abfiltriert oder das Reaktionsgemisch kann über eine Ionenaustauscherschüttung geleitet werden.

Bevorzugt wird der Veresterungskatalysator im Reaktionsgemisch belassen.

Als Lösungsmittel zur azeotropen Entfernung des Reaktionswassers eignen sich vor allem aliphati-sche, cycloaliphatische und aromatische Kohlenwasser-stoffe oder Gemische davon.

Vorzugsweise kommen n-Pentan, n-Hexan, -Heptan, Cyclohexan, Methylcyclohexan, Benzol, Toluol oder Xylol zur Anwendung. Besonders bevorzugt sind Cyclohexan, Methylcyclohexan und Toluol.

Die eingesetzte Menge beträgt beispielsweise 10 - 200 Gew.-%, vorzugsweise 20 - 100 Gew.-%, besonders bevorzugt 30 - 100 Gew.-% bezogen auf die Summe von (Meth)acrylsäure und $C_{17}$-Alkoholgemisch.

Die Reaktionstemperatur liegt im Allgemeinen bei 60 - 140 °C, vorzugsweise 70 - 110 °C, ganz besonders bevorzugt bei 75 - 100 °C. Die Anfangstemperatur liegt allgemein unter 100 °C, bevorzugt unter 90 °C und besonders bevorzugt unter 80 °C. In der Regel ist Endtemperatur der Veresterung um 5 - 30 °C höher als die Anfangstemperatur. Die Temperatur der Veresterung kann durch Variation der Lösungsmittelkonzentration im Reaktionsgemisch bestimmt und geregelt werden, wie in DE 199 41 136 A1 und DE 100 63 175 A1 beschrieben.

Die Veresterung kann drucklos aber auch bei Überdruck oder Unterdruck durchgeführt werden, vorzugsweise wird bei normalem Druck gearbeitet.

Die Reaktionszeit beträgt in der Regel 30 Minuten bis 10 Stunden, vorzugsweise 1 - 6 und besonders bevorzugt 2 - 4 Stunden.

Die Zugabe der Edukte (Meth)acrylsäure und $C_{17}$-Alkoholgemisch, sowie der sonstigen Komponenten wie Lösungsmittel, Polymerisationsinhibitor(gemisch) und Katalysator kann beliebig erfolgen.

In einer bevorzugten Ausführungsform werden Lösungsmittel und das $C_{17}$-Alkoholgemisch zumindest teilweise, bevorzugt vollständig, im Reaktor vorgelegt und erhitzt. Sobald der Umlauf in Betrieb ist, können die restlichen Komponenten (Meth)acrylsäure, Polymerisationsinhibitor(gemisch) und Katalysator gemeinsam oder getrennt voneinander zudosiert werden. Die Zudosierung erfolgt in der Regel innerhalb von 0,5 - 5 Stunden kontinuierlich oder portionsweise.

Die einsetzbare (Meth)acrylsäure ist nicht beschränkt und kann im Fall von Roh-(Meth)acrylsäure beispielsweise folgende Komponenten aufweisen:

| | |
|---|---|
| (Meth)acrylsäure | 90 - 99,9 Gew.-% |
| Essigsäure | 0,05 - 3 Gew.-% |
| Propionsäure | 0,01 - 1 Gew.-% |
| Diacrylsäure | 0,01 - 5 Gew.-% |
| Wasser | 0,05 - 5 Gew.-% |
| Aldehyde | 0,01 - 0,3 Gew.-% |
| Inhibitoren | 0,01 - 0,1 Gew.-% |
| Maleinsäure(-anhydrid) | 0,001 - 0,5 Gew.-% |

Die eingesetzte Roh-(Meth)acrylsäure ist in der Regel stabilisiert mit 100 - 600 ppm, bevorzugt mit 200 - 500 ppm eines der unten genannten Polymerisations-inhibitoren, bevorzugt Phenothiazin oder Hydrochinon-monomethylether oder anderen Stabilisatoren, die eine vergleichbare Stabilisierung ermöglichen.

Selbstverständlich kann auch Rein-(Meth)acrylsäure eingesetzt werden mit beispielsweise folgender Reinheit:

| | |
|---|---|
| (Meth)acrylsäure | 99,7 - 99,99 Gew.-% |
| Essigsäure | 50 - 1000 Gew.-ppm |
| Propionsäure | 10 - 500 Gew.-ppm |
| Diacrylsäure | 10 - 500 Gew.-ppm |
| Wasser | 50 - 1000 Gew.-ppm |
| Aldehyde | 1 - 500 Gew.-ppm |

(fortgesetzt)

| Inhibitoren | 1 - 300 Gew.-ppm |
| Maleinsäure(-anhydrid) | 1 - 200 Gew.-ppm |

Die eingesetzte Rein-(Meth)acrylsäure ist in der Regel stabilisiert mit 100 - 400 ppm, bevorzugt mit 200 - 300 ppm eines der unten genannten Polymerisationsinhibitoren, bevorzugt Phenothiazin oder Hydrochinonmonomethylether oder anderen Stabilisatoren, die eine vergleichbare Stabilisierung ermöglichen.

Das bei der Reaktion gebildete Wasser wird als Azeotrop mit dem Lösungsmittel kontinuierlich über die dem Reaktor aufgesetzte Kolonne aus dem Reaktionsgemisch entfernt und kondensiert, wobei das Kondensat in eine Wasser- und eine organische Phase zerfällt.

Die wässrige Phase des Kondensats, die in der Regel 0,1 - 10 Gew.-% (Meth)acrylsäure enthält, wird abgetrennt und ausgeschleust. Vorteilhafterweise kann die darin enthaltene (Meth)acrylsäure mit einem Extraktionsmittel, beispielsweise mit Cyclohexan, bei einer Temperatur zwischen 10 und 40 °C und einem Verhältnis von wässriger Phase zu Extraktionsmittel von 1 : 5 - 30, bevorzugt 1 : 10 - 20, extrahiert und in die Veresterung zurückgeführt werden.

Die organische Phase kann ganz oder teilweise als Rücklauf in die Kolonne und der ggf. überschüssige Rest kann in den Reaktor zurückgeführt werden. Ein Teil dieser Phase kann bei Verwendung eines Naturumlaufs ggf. zur Unterstützung des Naturumlaufs in den Wärmetauscher des Umlaufkreislaufs des Reaktors eingebracht werden, bevorzugt mindestens 10 Gew.-% der organischen Phase, besonders bevorzugt mindestens 15 Gew.-% und ganz besonders bevorzugt mindestens 20 Gew.-%.

Eine vorteilhafte Variante besteht darin, dass die organische Phase (Lösungsmittelphase) in einen Vorratsbehälter geleitet wird und dass aus diesem Behälter die jeweils erforderliche Lösungsmittelmenge zur Aufrechterhaltung des Rücklaufs, zum Einleiten in den Umlaufverdampfer, sowie als Lösungsmittel für Reaktion und Extraktion entnommen wird.

Zur weiteren Unterstützung des Umlaufs kann ein inertes Gas, bevorzugt ein sauerstoffhaltiges Gas, besonders bevorzugt Luft oder ein Gemisch aus Luft und Stickstoff (Magerluft) in den Umlauf geleitet werden, beispielsweise in Mengen von 0,1 - 1, bevorzugt 0,2 - 0,8 und besonders bevorzugt 0,3 - 0,7 m$^3$/m$^3$h, bezogen auf das Volumen des Reaktionsgemisches.

Der Verlauf der Veresterung kann durch Verfolgung der ausgetragenen Wassermenge und/oder die Abnahme der (Meth)acrylsäurekonzentration im Reaktor verfolgt werden.

Die Reaktion kann beispielsweise beendet werden, sobald 90 % der theoretisch zu erwartenden Wassermenge durch das Lösungsmittel ausgetragen worden sind, bevorzugt bei mindestens 95 % und besonders bevorzugt bei mindestens 98 %.

Nach Beendigung der Veresterung wird das Reaktionsgemisch auf übliche Weise rasch auf eine Temperatur von 10 bis 30 °C abgekühlt, gegebenenfalls durch Zugabe von Lösungsmittel eine Zielesterkonzentration von 60 - 80 % eingestellt.

2) Vorwäsche und Neutralisation

Das Reaktionsgemisch wird gegebenenfalls in einem Waschapparat mit Wasser oder einer 5 - 30 Gew.-%igen, bevorzugt 5 - 20, besonders bevorzugt 5 - 15 Gew.-%igen Kochsalz-, Kaliumchlorid-, Ammoniumchlorid-, Natriumsulfat- oder Aluminiumsulfatlösung, bevorzugt Kochsalzlösung behandelt.

Das Mengenverhältnis Reaktionsgemisch : Waschflüssigkeit beträgt in der Regel 1 : 0,1 - 1, bevorzugt 1 : 0,2 - 0,8, besonders bevorzugt 1 : 0,3 - 0,7.

Die Wäsche kann beispielsweise in einem Rührbehälter oder in einer anderen herkömmlichen Apparatur, z. B. in einer Kolonne oder Mixer-Settler-Apparatur durchgeführt werden.

Verfahrenstechnisch können für eine Wäsche im erfindungsgemäßen Verfahren alle an sich bekannten Extraktions- und Waschverfahren und -apparate eingesetzt werden, z. B. solche, die in Ullmann's Encyclopedia of Industrial Chemistry, 6th ed, 1999 Electronic Release, Kapitel: Liquid - Liquid Extraction - Apparatus, beschrieben sind. Beispielsweise können dies ein- oder mehrstufige, bevorzugt einstufige Extraktionen, sowie solche in Gleich- oder Gegenstromfahrweise sein.

Die Vorwäsche wird bevorzugt dann eingesetzt, wenn Metallsalze, bevorzugt Kupfer oder Kupfersalze als Inhibitoren (mit)verwendet werden.

Die organische Phase der Vorwäsche, die noch geringe Mengen an Katalysator und die Hauptmenge an überschüssiger (Meth)acrylsäure enthält, wird mit einer 5 - 25, bevorzugt 5 - 20, besonders bevorzugt 5 - 15 Gew.-% wässrigen Lösung einer Base, wie z.B. Natronlauge, Kalilauge, Natriumhydrogencarbonat, Natriumcarbonat, Kaliumhydrogencarbonat, Calciumhydroxid, Ammoniakwasser oder Kaliumcarbonat, der gegebenenfalls 5 - 15 Gew.-% Kochsalz, Kaliumchlorid, Ammoniumchlorid oder Ammoniumsulfat zugesetzt sein können, bevorzugt mit Natronlauge oder Natronlauge-Kochsalz-Lösung neutralisiert.

Die Zugabe der Base erfolgt in einer Weise, dass die Temperatur im Apparat nicht über 35 °C ansteigt, bevorzugt zwischen 20 und 35 °C beträgt und der pH-Wert 10 - 14 beträgt. Die Abfuhr der Neutralisationswärme erfolgt gegebenenfalls durch Kühlung des Behälters mit Hilfe von innenliegenden Kühlschlangen oder über eine Doppelwandkühlung.

Das Mengenverhältnis Reaktionsgemisch : Neutralisationsflüssigkeit beträgt in der Regel 1 : 0,1 - 1, bevorzugt 1 : 0,2 - 0,8, besonders bevorzugt 1 : 0,3 - 0,7.

Hinsichtlich der Apparatur gilt das oben Gesagte.

Optional kann zur Entfernung von Base- oder Salzspuren aus dem neutralisierten Reaktionsgemisch eine Nachwäsche vorteilhaft sein, welche analog zur Vorwäsche durchgeführt werden kann.

3) Lösungsmitteldestillation

Das gewaschene Reaktionsgemisch wird mit einer derartigen Menge an Lagerstabilisator, bevorzugt Hydrochinonmonomethylether versetzt, dass nach Abtrennung des Lösungsmittels 100 - 500, bevorzugt 200 - 500 und besonders bevorzugt 200 - 400 ppm davon im Zielester enthalten sind.

Die destillative Abtrennung der Hauptmenge an Lösungsmittel erfolgt beispielsweise in einem Rührkessel mit Doppelwandheizung und/oder innenliegenden Heizschlangen unter vermindertem Druck, beispielsweise bei 20 - 700 mbar, bevorzugt 30 bis 500 und besonders bevorzugt 50 - 150 mbar und einer Temperatur von 40 - 80 °C.

Selbstverständlich kann die Destillation auch in einem Fallfilm- oder Dünnschichtverdampfer erfolgen. Dazu wird das Reaktionsgemisch, bevorzugt mehrmals im Kreislauf, unter vermindertem Druck, beispielsweise bei 20 - 700 mbar, bevorzugt 30 bis 500, besonders bevorzugt 50 - 150 mbar und einer Temperatur von 40 - 80 °C durch den Apparat geführt.

Vorteilhaft kann ein inertes Gas, bevorzugt ein sauerstoffhaltiges Gas, besonders bevorzugt Luft oder ein Gemisch aus Luft und Stickstoff (Magerluft) in den Destillationsapparat eingeleitet werden, beispielsweise 0,1 - 1, bevorzugt 0,2 - 0,8 und besonders bevorzugt 0,3 - 0,7 $m^3/m^3h$, bezogen auf das Volumen des Reaktionsgemisches.

Der Restlösungsmittelgehalt im Rückstand beträgt nach der Destillation in der Regel unter 5 Gew.-%, bevorzugt 0,5 - 5 Gew.-%.

Das abgetrennte Lösungsmittel wird kondensiert und bevorzugt wieder verwendet.

(Meth)acrylsäure und (Meth)acrylsäureester von $C_{17}$-Alkoholgemischen sind polymerisationsfähige Verbindungen. Daher ist in allen Verfahrensschritten auf eine ausreichende Polymerisationsinhibierung zu achten. Eine unerwünschte Polymerisation ist aufgrund der freiwerdenden großen Wärmemenge sicherheitstechnisch bedenklich.

Daher erfolgen beim erfindungsgemäßen Verfahren sowohl die Veresterungsreaktion als auch die thermischen Trennungen vorzugsweise in Gegenwart von üblichen Mengen an sich bekannter Polymerisationsinhibitoren. In der Regel werden, bezogen auf die $\alpha,\beta$-monoethylenisch ungesättigten Monomeren, je Einzelsubstanz von 1 bis 10 000 ppm, bevorzugt von 10 bis 5 000 ppm, besonders bevorzugt von 30 bis 2 500 ppm und insbesondere von 50 bis 1 500 ppm eines geeigneten Stabilisators eingesetzt.

Geeignete Stabilisatoren können beispielsweise N-Oxide (Nitroxyl- oder N-Oxyl-Radikale, also Verbindungen, die wenigstens eine >N-O.-Gruppe aufweisen), wie z. B. 4-Hydroxy-2,2,6,6-tetramethylpiperidin-N-oxyl, 4-Oxo-2,2,6,6-tetramethylpiperidin-N-oxyl, 4-Acetoxy-2,2,6,6-tetramethyl-piperidin-N-oxyl, 2,2,6,6-Tetramethylpiperidin-N-oxyl, Bis(1-oxyl-2,2,6,6-tetramethylpiperidine-4-yl)sebacat, 4,4',4"-Tris(2,2,6,6-tetramethyl-piperidin-N-oxyl)-phosphit oder 3-Oxo-2,2,5,5-tetramethyl-pyrrolidin-N-oxyl; ein- oder mehrwertige Phenole, die ggf. eine oder mehrere Alkylgruppen aufweisen, wie z. B. Alkylphenole, beispielsweise o-, m- oder p-Kresol (Methylphenol), 2-tert.-Butylphenol, 4-tert.-Butylphenol, 2,4-Di-tert.-butylphenol, 2-Methyl-4-tert.-butylphenol, 2-tert.-Butyl-4-methylphenol, 2,6-tert.-Butyl-4-methylphenol, 4-tert.-Butyl-2,6-dimethylphenol oder 6-tert.-Butyl-2,4-dimethylphenol; Chinone, wie z. B. Hydrochinon, Hydrochinonmonomethylether, 2-Methylhydrochinon oder 2,5-Di-tert.-Butylhydrochinon; Hydroxyphenole, wie beispielsweise Brenzcatechin (1,2-Dihydroxybenzol) oder Benzochinon; Aminophenole, wie z. B. p-Aminophenol; Nitrosophenole, wie z. B. p-Nitrosophenol; Alkoxyphenole, wie beispielsweise 2-Methoxyphenol (Guajacol, Brenzcatechinmonomethylether), 2-Ethoxyphenol, 2-Isopropoxyphenol, 4-Methoxyphenol (Hydrochinonmonomethylether), Mono- oder Di-tert.-Butyl-4-methoxyphenol; Tociperole, wie z. B. α-Tocopherol sowie 2,3-Dihydro-2,2-dimethyl-7-hydroxybenzofuran (2,2-Dimethyl-7-hydroxycumaran), aromatische Amine, wie z. B. N,N-Diphenylamin oder N-Nitroso-diphenylamin; Phenylendiamine, wie z. B. N,N'-Dialkyl-p-phenylendiamin, wobei die Alkylreste gleich oder verschieden sein können und jeweils unabhängig voneinander aus 1 bis 4 Kohlenstoffatomen bestehen und geradkettig oder verzweigt sein können, wie z. B. N,N'-Dimethyl-p-phenylendiamin oder N,N'-Diethyl-p-phenylendiamin, Hydroxylamine, wie z.B. N,N-Diethylhydroxylamin, Imine, wie z. B. Methylethylimin oder Methylen violett, Sulfonamide, wie z. B. N-Methyl-4-toluolsulfonamid oder N-tert.-Butyl-4-toluolsulfonamid, Oxime, wie Aldoxime, Ketoxime oder Amidoxime, wie z. B. Diethylketoxim, Methylethylketoxim oder Salicyladoxim, phosphorhaltige Verbindungen, wie z. B. Triphenylphosphin, Triphenylphosphit, Triethylphosphit, Hypophsophorige Säure oder Alkylester der Phosphorigen Säuren; schwefelhaltige Verbindungen wie z. B. Diphenylsulfid oder Phenothiazin; Metallsalze, wie Kupfer- oder Mangan-, Cer-, Nickel-, Chromsalze, beispielsweise -chloride, - sulfate, -salicylate, -tosylate, -acrylate oder -acetate, wie z. B. Kupferacetat, Kupfer(II)chlorid, Kupfersalicylat, Cer(III)acetat oder Cer(III)ethylhexanoat, oder Gemische davon sein.

Bevorzugt wird als Polymerisationsinhibitor(gemisch) mindestens eine Verbindung aus der Gruppe Hydrochinon, Hydrochinonmonomethylether, Phenothiazin, 4-Hydroxy-2,2,6,6-tetramethylpiperidin-N-oxyl, 4-Oxo-2,2,6,6-tetramethylpiperidin-N-oxyl, Bis(1-oxyl-2,2,6,6-tetramethylpiperidine-4-yl)sebacat, 2-tert.-Butylphenol, 4-tert.-Butylphenol, 2,4-Di-tert.-Butylphenol, 2-tert.-Butyl-4-methylphenol, 6-tert.-Butyl-2,4-dimethylphenol, 2,6-Di-tert.-Butyl-4-methylphenol, 2-Methyl-4-tert.-butylphenol, Hypophosphorige Säure, Kupferacetat, Kupfer(II)chlorid, Kupfersalicylat und Cer(III)acetat.

Ganz besonders bevorzugt wird Phenothiazin und/oder Hydrochinonmonomethylether (MEHQ) als Polymerisationsinhibitor verwendet.

Bevorzugt wird der/das Polymerisationsinhibitor(gemisch) als wässrige Lösung eingesetzt.

Zur weiteren Stützung der Stabilisierung kann ein sauerstoffhaltiges Gas, bevorzugt Luft oder ein Gemisch aus Luft und Stickstoff (Magerluft) anwesend sein.

Im Verfahrensschritt der Veresterung wird das sauerstoffhaltige Gas vorzugsweise in den Sumpfbereich der Kolonne und/oder in einen Umlaufverdampfer eindosiert.

Die erfindungsgemäß hergestellten (Meth)acrylate von $C_{17}$-Alkoholgemischen finden Anwendung beispielsweise als Monomere oder Comonomere in der Herstellung von Dispersionen, die u.a. als Klebstoffe, Anstrichmittel oder Textil-, Leder- und Papierhilfsmittel eingesetzt werden.

Darüber hinaus können die nach dem erfindungsgemäßen Verfahren hergestellten (Meth)acrylate von $C_{17}$-Alkoholgemischen als Comonomer in Polymeren Anwendung finden, die wiederum als Additiv für Brennstofföle und Schmierstoffe und insbesondere als Kaltfließverbesserer in Brennstoffölen eingesetzt werden. Eine derartige Verwendung ist beispielsweise in der europäischen Anmeldung EP 1 923 454 A1 offenbart.

Das folgende Beispiel soll die Eigenschaften der Erfindung erläutern, ohne sie aber einzuschränken.

Falls nicht anders angegeben, bedeuten Prozent immer Gewichtsprozent und Teile immer Gewichtsteile.

US Provisional Patent Application No. 61/264,704, eingereicht am 27. November 2009, ist in die vorliegende Anmeldung eingefügt durch Literaturhinweis. Im Hinblick auf die obengenannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

Beispiel

In einer Veresterungsapparatur (2L-4-Halskolben mit Innenthermometer, Rückflusskühler und Wasserabscheider) wurde die Veresterung von Acrylsäure mit einem $C_{17}$-Alkoholgemisch durchgeführt. Dabei wurden 800 ml Cyclohexan, 513 g (2,0 mol) Heptadecanol (mittlerer Verzweigungsgrad von ca. 3,0) und 3,0 ml Stabilisatorlösung (1,25 g Hydrochinonmono-methylether (MEHQ) und 3,25 g Hypophosphorige Säure gelöst in 37,5 g Wasser), 1,2 ml 20 %ige Kupfer(II)chloridlösung vorgelegt und 180,3 g (2,5 mol) Acrylsäure (stabilisiert mit 200 ppm MEHQ) zugegeben. Das Gemisch wurde unter Luft-Atmosphäre erhitzt, und bei einer Innentemperatur von 75 °C wurden 9,6 ml 98 %ige Methansulfonsäure zugegeben. Nach 2 Stunden Sieden unter Rückfluss, wobei kontinuierlich Wasser abgetrennt wurde, wurde die Reaktionslösung abgekühlt.

Zu der entstandenen klaren Lösung wurden 240 ml 7,5 %ige Natriumchloridlösung gegeben. Mit 160 ml einer 12,5 %igen Natronlauge wurde ein pH-Wert von 13 eingestellt. Nach dem Ausschütteln wurde die Cyclohexanphase abgetrennt und noch zweimal mit je 240 ml 7,5 %iger Natriumchloridlösung gewaschen, über Natriumsulfat getrocknet, filtriert und mit 122 mg (200 ppm) MEHQ versetzt. Anschließend wurde das Lösungsmittel im Vakuum entfernt. Es wurde eine klare Flüssigkeit erhalten.

Man erhielt Heptadecylacrylat in einer Ausbeute von 552,4 g (89 %) und einer Reinheit > 95 % sowie einer APHA-Farbzahl von 9.

**Patentansprüche**

1. Verfahren zur Herstellung von (Meth)acrylaten von $C_{17}$-Alkoholgemischen, wobei der Erstarrungspunkt der (Meth)acrylate bei Atmospärendruck unter 0 °C liegt, durch Umsetzung von (Meth)acrylsäure mit einem $C_{17}$-Alkoholgemisch in Gegenwart mindestens eines sauren Katalysators und mindestens eines Polymerisationsinhibitors und in Gegenwart eines Lösungsmittels, das mit Wasser ein Azeotrop bildet, in dem die Veresterung in einem Reaktor mit Umlaufverdampfer durchführt wird, das Azeotrop abdestilliert und kondensiert wird, das Kondensat in eine wässrige und eine organische Phase zerfällt, **dadurch gekennzeichnet, dass** das $C_{17}$-Alkoholgemisch einen mittleren Verzweigungsgrad (Iso-Index) von 3,01 bis 3,5 und einen Gehalt an Alkoholen mit 17 Kohlenstoffatomen von mindestens 95 Gew.-% aufweist.

2. Verfahren nach Anspruch 1, **dadurch gekennzeichnet, dass** das $C_{17}$-Alkoholgemisch einen mittleren Verzweigungsgrad 3,05 bis 3,4 aufweist.

3. Verfahren nach Anspruch 1 oder 2, **dadurch gekennzeichnet, dass** das $C_{17}$-Alkoholgemisch einen mittleren Verzweigungsgrad im Bereich von etwa 3,1 aufweist.

4. Verfahren nach einem der vorstehenden Ansprüche, **dadurch gekennzeichnet, dass** das $C_{17}$-Alkoholgemisch einen Gehalt an Alkoholen mit 17 Kohlenstoffatomen von mindestens 98 Gew.-% aufweist.

5. Verfahren nach einem der vorstehenden Ansprüche, **dadurch gekennzeichnet, dass** als saure Katalysatoren Mineralsäuren, Sulfonsäuren oder Gemische davon eingesetzt werden.

6. Verfahren nach einem der vorstehenden Ansprüche, **dadurch gekennzeichnet, dass** als Polymerisati-

**EP 2 504 308 B1**

onsinhibitor(gemisch) mindestens eine Verbindung aus der Gruppe Hydrochinon, Hydrochinonmonomethylether, Phenothiazin, 4-Hydroxy-2,2,6,6-tetramethylpiperidin-N-oxyl, 4-Oxo-2,2,6,6-tetramethyl-piperidin-N-oxyl, Bis(1-oxyl-2,2,6,6-tetramethylpiperidine-4-yl)sebacat, 2-tert.-Butylphenol, 4-tert.-Butylphenol, 2,4-Di-tert.-Butylphenol, 2-tert.-Butyl-4-methylphenol, 6-tert.-Butyl-2,4-dimethylphenol, 2,6-Di-tert.-Butyl-4-methylphenol, 2-Methyl-4-tert.-butylphenol, Hypophosphorige Säure, Kupferacetat, Kupfer(II)chlorid, Kupfersalicylat und Cer(III)acetat eingesetzt wird.

7. Verwendung von (Meth)acrylsäureester hergestellt gemäß dem Verfahren nach einem der vorstehenden Ansprüche 1 bis 6 als Monomere oder Comonomere in der Herstellung von Dispersionen.

8. Verwendung von Dispersionen hergestellt nach Anspruch 7, **dadurch gekennzeichnet, dass** die Dispersionen als Klebstoffe, Anstrichmittel, Textil-, Leder- oder Papierhilfsmittel oder als Additiv für Brennstofföle und Schmierstoffe eingesetzt werden.

**Claims**

1. A process for preparing (meth) acrylates of $C_{17}$ alcohol mixtures, where the solidification point of the (meth)acrylates at atmospheric pressure is below 0°C, by reacting (meth)acrylic acid with a $C_{17}$ alcohol mixture in the presence of at least one acidic catalyst and of at least one polymerization inhibitor and in the presence of a solvent which forms an azeotrope with water, in which the esterification is performed in a reactor with a circulation evaporator, the azeotrope is distilled off and condensed, and the condensate splits into an aqueous phase and an organic phase, wherein the $C_{17}$ alcohol mixture has a mean degree of branching (iso index) of 3.01 to 3.5 and has a content of alcohols having 17 carbon atoms of at least 95% by weight.

2. The process according to claim 1, wherein the $C_{17}$ alcohol mixture has a mean degree of branching of 3.05 to 3.4.

3. The process according to claim 1 or 2, wherein the $C_{17}$ alcohol mixture has a mean degree of branching of about 3.1.

4. The process according to any of the preceding claims, wherein the $C_{17}$ alcohol mixture has a content of alcohols having 17 carbon atoms of at least 98% by weight.

5. The process according to any of the preceding claims, wherein the acidic catalysts used are mineral acids, sulfonic acids or mixtures thereof.

6. The process according to any of the preceding claims, wherein the polymerization inhibitor (mixture) used is at least one compound from the group of hydroquinone, hydroquinone monomethyl ether, phenothiazine, 4-hydroxy-2,2,6,6-tetramethylpiperidine N-oxyl, 4-oxo-2,2,6,6-tetramethylpiperidine N-oxyl, bis(1-oxyl-2,2,6,6-tetramethyl-piperidin-4-yl) sebacate, 2-tert-butylphenol, 4-tert-butylphenol, 2,4-di-tert-butylphenol, 2-tert-butyl-4-methylphenol, 6-tert-butyl-2,4-dimethylphenol, 2,6-di-tert-butyl-4-methylphenol, 2-methyl-4-tert-butylphenol, hypophosphorous acid, copper acetate, copper (II) chloride, copper salicylate and cerium(III) acetate.

7. The use of (meth) acrylic esters obtainable by the process according to any of the preceding claims 1 to 6 as monomers or comonomers in the preparation of dispersions.

8. The use of dispersions prepared according to claim 7, wherein the dispersions are used as adhesives, paints, textile, leather or papermaking assistants, or as additive for fuel oils and lubricants.

**Revendications**

1. Procédé pour la préparation de (méth)acrylates de mélanges d'alcools en $C_{17}$, le point de solidification des (méth)acrylates à la pression atmosphérique se situant en dessous de 0 °C, par transformation d'acide (méth)acrylique avec un mélange d'alcools en $C_{17}$ en présence d'au moins un catalyseur acide et d'au moins un inhibiteur de polymérisation et en présence d'un solvant, qui forme un azéotrope avec l'eau, dans lequel l'estérification est mise en œuvre dans un réacteur doté d'un évaporateur à circulation, l'azéotrope est distillé et condensé, le condensat se désagrège dans une phase aqueuse et une phase organique, **caractérisé en ce que** le mélange d'alcools en $C_{17}$ présente un degré moyen de ramification (indice iso) de 3,01 à 3,5 et une teneur en alcools comportant 17 atomes de carbone d'au moins 95 % en poids.

2. Procédé selon la revendication 1, **caractérisé en ce que** le mélange d'alcools en $C_{17}$ présente un degré moyen de ramification de 3,05 à 3,4.

3. Procédé selon la revendication 1 ou 2, **caractérisé en ce que** le mélange d'alcools en $C_{17}$ présente un degré moyen de ramification dans la plage d'environ 3,1.

4. Procédé selon l'une quelconque des revendications précédentes, **caractérisé en ce que** le mélange

8

d'alcools en $C_{17}$ présente une teneur en alcools comportant 17 atomes de carbone d'au moins 98 % en poids.

5. Procédé selon l'une quelconque des revendications précédentes, **caractérisé en ce que** des acides minéraux, des acides sulfoniques ou des mélanges de ceux-ci sont utilisés en tant que catalyseurs acides.

6. Procédé selon l'une quelconque des revendications précédentes, **caractérisé en ce qu'**au moins un composé du groupe de l'hydroquinone, du monométhyléther d'hydroquinone, de la phénothiazine, du 4-hydroxy-2,2,6,6-tétraméthylpipéridin-N-oxyle, du 4-oxo-2,2,6,6-tétraméthylpipéridin-N-oxyle, du sébaçate de bis(1-oxyl-2,2,6,6-tétraméthylpipéridin-4-yle, du 2-tert.-butyl-phénol, du 4-tert.-butyl-phénol, du 2,4-di-tert.-butyl-phénol, du 2-tert.-butyl-4-méthylphénol, du 6-tert.-butyl-2,4-diméthylphénol, du 2,6-di-tert.-butyl-4-méthylphénol, du 2-méthyl-4-tert.-butyl-phénol, de l'acide hypophosphoreux, de l'acétate de cuivre, du chlorure de cuivre(II), du salicylate de cuivre et de l'acétate de cérium(III) est utilisé en tant qu'inhibiteur de polymérisation ou en tant que mélange d'inhibiteur de polymérisation.

7. Utilisation d'esters d'acide (méth)acrylique préparés selon le procédé selon l'une quelconque des revendications précédentes 1 à 6 en tant que monomères ou comonomères dans la préparation de dispersions.

8. Utilisation de dispersions préparées selon la revendication 7, **caractérisée en ce que** les dispersions sont utilisées en tant qu'adhésifs, peintures, adjuvants pour textile, cuir ou papier ou en tant qu'additif pour huiles combustibles et lubrifiants.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2002050014 A1
- WO 2002050015 A1
- DE 2317226 A1
- JP 11080082 A
- JP 5070404 A

- WO 2009124979 A1
- DE 19941136 A1
- DE 10063175 A1
- EP 1923454 A1
- US 61264704

### In der Beschreibung aufgeführte Nicht-Patentliteratur

- **R. BILLET.** Verdampfertechnik. HTB-Verlag, Bibliographisches Institut Mannheim, 1965, 53